# EUROPÄISCHE PATENTSCHRIFT

(11) **EP 0 900 190 B1**
(45) Veröffentlichungstag und Bekanntmachung des Hinweises auf die Patenterteilung: **24.10.2001**
(21) Anmeldenummer: 97920683.6
(22) Anmeldetag: 15.04.1997
(51) Int. Cl.: C07C 69/708, C07C 69/734, C07C 67/31, C07C 235/08, C07C 59/125, C07D 295/185, C10M 105/38, C10M 105/68

(54) **NEUE ACETALE VON HYDROXYCARBONSÄUREN UND DEREN DERIVATEN UND VERFAHREN ZU IHRER HERSTELLUNG**
NOVEL ACETALS OF HYDROXY CARBOXYLIC ACIDS, DERIVATIVES THEREOF AND PROCESS FOR THE PREPARATION THEREOF
NOUVEAUX ACETALS D'ACIDES HYDROXYCARBOXYLIQUES ET LEURS DERIVES ET PROCEDE PERMETTANT DE LES PREPARER

(30) Priorität: 24.04.1996 DE 19616339
(43) Veröffentlichungstag der Anmeldung: 10.03.1999
(73) Patentinhaber: Clariant GmbH, 65929 Frankfurt am Main (DE)
(72) Erfinder: KLUG, Peter, D-63762 Gro ostheim (DE); WEINELT, Frank, D-84508 Burgkirchen (DE); FALK, Uwe, D-63486 Bruchköbel (DE)
(86) Internationale Anmeldenummer: EP9701872
(87) Internationale Veröffentlichungsnummer: WO9740005

(56) Entgegenhaltungen:
- US-A- 2 327 116
- JOURNAL OF ORGANIC CHEMISTRY, Bd. 38, Nr. 13, 29.Juni 1973, EASTON US, Seiten 2346-2350, XP002035962 PETER Y.JOHNSON ET AL.: "The Reformatsky Reaction of Ethyl alpha-Bromo Esters with Bis(chloromethyl)Ether"

## Beschreibung

Dimerfettsäurederivate stellen wichtige Produkte im Kunststoff-, Erdöl-, Schmierstoff- und Fasersektor dar (J. Am. Oil Chem. Soc. 1979, 56, A 782). Sie finden z. B. Verwendung als Weichmacherkomponenten, als Monomerbausteine für Polyamide, Tenside, Korrosionsinhibitoren und Faserpräparationsmittel. Sie zeichnen sich im allgemeinen trotz des hohen Molekulargewichtes dadurch aus, daß sie flüssig sind und vergleichsweise niedrige Viskositäten aufweisen. Weiterhin sind Dimerfettsäurederivate sehr temperaturstabil. Nachteilig für viele Anwendungen ist aber im Hinblick auf ökologische Gesichtspunkte deren ungenügende biologische Abbaubarkeit. Ein weiterer Nachteil besteht darin, daß zur Synthese von Dimerfettsäurederivaten relativ hohe Temperaturen von mehr als 200 °C nötig sind.

US-2 327 116 offenbart Diesteracetale, in denen die Esterfunktionen an die Acetalfunktion durch eine Methylenbrücke gebunden sind.

Somit bestand die Aufgabe, Stoffe zur Verfügung zu stellen, die die vorteilhaften Eigenschaften der Dimerfettsäurederivate aufweisen, im Gegensatz zu diesen jedoch bei niedrigeren Temperaturen herstellbar sind und nach der bestimmungsgemäßen Verwendung spaltbar und biologisch abbaubar sind.

Überraschenderweise wurde nun gefunden, daß Acetale der allgemeinen Formel R³CO-X-O-C(R¹R²)-O-Y-COR⁴, worin
- R¹, R²: unabhängig voneinander Wasserstoff, einen substituierten oder unsubstituierten C₁-C₁₂-Alkyl-, C₆-C₁₂-Aryl- oder C₇-C₁₂-Aralkylrest,
- X, Y: unabhängig voneinander einen verzweigten oder unverzweigten,substituierten oder unsubstituierten C₁₅-C₃₀-Alkylenrest oder einen verzweigten oder unverzweigten, substituierten oder unsubstituierten C₁₅-C₃₀-Alkenylenrest,
- R³, R⁴: unabhängig voneinander -OH, -OM, -OR⁵, -NH₂, -NHR⁶ oder -NR⁷ R⁸ bedeuten, wobei
- -M: ein Alkali-, Erdalkali- oder ein Ammoniumion der Formel HN⁺R⁹R¹⁰R¹¹ oder Mischungen davon bedeutet, wobei
- R⁹ bis R¹¹: unabhängig voneinander Wasserstoff, C₁-C₁₀-Alkyl oder C₁-C₁₀-Hydroxyalkyl bedeuten und wobei
- R⁵ bis R⁸: unabhängig voneinander einen C₁-C₂₀₀-Alkyl-, einen C₆-C₅₀-Aryl- oder einen C₇-C₅₀-Aralkylrest bedeuten, und wobei
R⁵ bis R⁸ mit Heteroatomen substituiert oder durch Heteroatome unterbrochen sein können und wobei
R⁷ und R⁸ gemeinsam einen Ring von 4 bis 10 Atomen bilden können,
diese vorteilhaften Eigenschaften aufweisen.

Diese Substanzen lassen sich aus einer C₂-C₃₁-Hydroxycarbonsäure, einem Ester oder einem Amid dieser Carbonsäure oder aus Mischungen davon, mit geeigneten Aldehyden oder Stoffen, die Aldehyde unter den Reaktionsbedingungen freisetzen, unter Entfernung von Reaktionswasser herstellen. Man erhält auf diese Weise Acetale mit dimersäureähnlicher Struktur, die sich von den Dimerfettsäuren durch ihre Kettenverknüpfung, die durch eine Acetalfunktion erfolgt, unterscheiden. Die Acetalfunktion ist oberhalb von pH 7 stabil, kann jedoch unter Rückbildung von Hydroxysäurederivaten nach Gebrauch im sauren pH-Bereich gespalten werden und ermöglicht so den biologischen Abbau des Produkts.

Die Substituenten R¹ und R² können dabei gesättigte oder ungesättigte, verzweigte oder unverzweigte, substituierte oder unsubstituierte C₁-C₁₂- Alkyl-reste oder C₆-C₁₂ Aryl- oder C₇-C₁₂ Aralkylreste sein. Auch Halogenatome können in diesen Resten enthalten sein. Besonders gut geeignete Substituenten sind Reste mit 1 bis 6 C-Atomen, insbesondere Reste ohne α-Wasserstoff wie Phenyl oder p-Nitrophenyl. Ganz besonders bevorzugt ist Wasserstoff.

X und Y können gesättigte, verzweigte oder unverzweigte, substituierte oder unsubstituierte C₁-C₃₀-Alkylenreste oder ungesättigte, verzweigte oder unverzweigte, substituierte oder unsubstituierte C₂-C₃₀-Alkenylenreste darstellen. Bevorzugt sind Reste mit 2 bis 20, ganz besonders bevorzugt sind Reste mit 15 bis 19 C-Atomen.

R³ und R⁴ können zusammen mit der Carbonylgruppe Säuregruppen oder deren Salze, Ester- oder Amingruppen darstellen. Bevorzugt sind Estergruppen -OR⁵ und Amingruppen -NHR⁶ oder -NR⁷R⁸.

R⁵ bis R⁸ können Alkylgruppen mit 1 bis 200, vor allem 1 bis 50, bevorzugt mit 1 bis 18, besonders bevorzugt mit 5 bis 13 C-Atomen oder Arylgruppen mit 6 bis 50 oder Aralkylgruppen mit 7 bis 50 C-Atomen darstellen. Bevorzugt sind Arylgruppen mit 6 bis 20, insbesondere mit 6 bis 10 C-Atomen sowie Aralkylgruppen mit 7 bis 20, insbesondere mit 7 bis 10 C-Atomen.
Dabei können diese Substituenten mit Heteroatomen wie F, Cl, I, O, N, S substituiert oder durch Heteroatome wie O, S oder N unterbrochen sein, wobei R⁷ und R⁸ gemeinsam einen Cyclus oder Heterocylus von 4 bis 10 Atomen bilden können. Besonders bevorzugt sind cyclische oder heterocyclische Verbindungen mit 6 Atomen im Ringgerüst.
R⁹ bis R¹¹ können Alkyl- oder Hydroxyalkylgruppen mit 1 bis 10, bevorzugt 1 bis 5 C-Atomen sein. Besonders bevorzugt ist eine Hydroxyalkylgruppe mit 2 C-Atomen.

Für die Herstellung der erfindungsgemäßen Acetale sind gesättigte und ungesättigte C₂-C₃₁-Hydroxycarbonsäuren mit primärer oder sekundärer Hydroxylgruppe wie Glykolsäure, Milchsäure, 4-Hydroxybutansäure bzw. y-Butyrolacton, 6-Hydroxyhexansäure bzw. Caprolacton, 12-Hydroxystearinsäure oder Ricinolsäure oder Hydroxyfettsäuren, die durch Oxidation von ungesättigten Fettsäuren gewonnen werden, geeignet. Insbesondere geeignet sind 12-Hydroxy-stearinsäure und Ricinolsäure und deren technische Qualitäten, die z. B. aus Ricinusöl gewonnen werden.
Neben diesen Hydroxyfettsäuren können auch deren Ester, Amide oder Mischungen aus diesen drei Komponenten in die entsprechenden Acetale überführt werden.

Die Darstellung von Hydroxyfettsäureestern ist im wesentlichen in der Literatur beschrieben; als Beispiele seien Ind. Eng. Chem. 1953, 45, 1777 sowie US-2,397,008, US 2,385,849 und US 2,390,027 genannt. Die Kondensation der Hydroxyfettsäure mit dem entsprechenden Alkohol wird dabei bevorzugt unter Säurekatalyse und azeotroper Entfernung des Reaktionswassers mit einem geeigneten Lösungsmittel bei 40 - 140 °C, insbesondere 60 - 110 °C, durchgeführt.

Geeignete Lösungsmittel sind z.B. aliphatische und aromatische, acyclische und cyclische Kohlenwasserstoffe, wie Petroleumbenzin, Cyclohexan, Toluol oder Xylol. Als Alkoholkomponente eignen sich aliphatische, cycloaliphatische oder aromatische Monoalkohole, deren Alkylketten durch weitere Heteroatome, insbesondere Sauerstoffatome, unterbrochen sein können.

Geeignete Alkohole sind z. B. Methanol, Ethanol, n-Propanol, Isopropanol, Butanole, Pentanole, 2-Ethylhexanol, Oleylalkohol, Stearylalkohol, Cyclohexanol, Benzylalkohol, Alkylpolyglykole wie Methylglykol, Methyldiglykol, Butyldiglykol, Methyltriglykol und Methyltetraglykol, oder ethoxilierter Oleylalkohol (z.B. Genapol® 0 050).

Geeignete Katalysatoren für die Veresterung sind starke Sulfonsäuren oder Mineralsäuren wie z. B. p-Toluolsulfonsäure, Methansulfonsäure, Schwefelsäure, Phosphorsäure sowie saure lonenaustauscher. Besonders bevorzugt sind Schwefelsäure und Methansulfonsäure in Konzentrationen von 0,01 - 2, vorzugsweise 0,1 - 1,0 Gew.-% bezüglich der Gesamtmasse von Alkohol und Hydroxyfettsäure.
Die Darstellung von Hydroxycarbonsäureamiden erfolgt aus Hydroxycarbonsäure und Amin mit oder ohne Katalysator durch Erhitzen der Komponenten in Substanz auf 150 - 220 °C bzw. unter azeotroper Entfernung von Reaktionswasser mit einem geeigneten Lösungsmittel bei 100 - 220 °C, insbesondere 150 - 200 °C.
Geeignete Lösungsmittel sind z.B. aliphatische und aromatische, acyclische und cyclische Kohlenwasserstoffe, wie Petroleumbenzin, Cyclohexan, Toluol oder Xylol.
Geeignete Amine sind z.B. aliphatische, cyclische und aromatische Amine, deren Alkylrest ggf. weitere Heteroatome enthält. Beispiele sind primäre Amine wie Methylamin, Methoxypropylamin, Butylamine, Oleylamin und sekundäre Amine wie Dimethylamin, Diethylamin, Dibutylamin, Morpholin, Piperidin und Pyrollidin.

Die Acetalisierung der Hydroxyfettsäuren und Hydroxyfettsäurederivate mit dem entsprechenden Aldehyd wird unter Säurekatalyse mit und ohne Lösungsmittel, bevorzugt unter azeotroper Entfernung des Reaktionswassers mit einem geeigneten Lösungsmittel bei 40 - 140 °C durchgeführt.

Als Aldehydkomponente geeignet sind aliphatische Aldehyde wie Formaldehyd, Acetaldehyd, Trichloracetaldehyd, Propionaldehyd, Butyraldehyd, Isobutyraldehyd, Isononylaldehyd und Dodecanal, aromatische Aldehyde wie Benzaldehyd und Naphthaldehyd und Verbindungen, die solche Aldehyde unter den Reaktionsbedingungen freisetzen können, wie z. B. Trioxan oder Paraformaldehyd, die trimeren und tetrameren Formen des Acetaldehyds sowie Dialkylacetale. Besonders geeignet sind Benzaldehyd oder p-Nitrobenzaldehyd, insbesondere geeignet sind Formaldehyd oder Paraformaldehyd, die vollständige Umsätze zum Acetal ermöglichen. Diese Aldehyde werden in nahezu äquimolarer oder überstöchiometrischer Menge, der Überschuß ist unkritisch, insbesondere zu 90 - 130 Gew.-% der theoretischen Menge eingesetzt.

Geeignete Lösungsmittel sind gesättigte und aromatische, acyclische und cyclische Kohlenwasserstoffe, wie Petroleumbenzin, Cyclohexan, Toluol oder Xylol.

Geeignete Katalysatoren für die Acetalisierung sind z. B. p-Toluolsulfonsäure, Methansulfonsäure, Schwefelsäure, Phosphorsäure sowie saure lonenaustauscher. Besonders bevorzugt sind Schwefelsäure und Methansulfonsäure in Konzentrationen von 0,01 - 2, bevorzugt 0,1 - 1,0 Gew.-% bezüglich des eingesetzten Hydroxyfettsäurederivats.

Die stattfindende Acetalbildung läßt sich durch die abgeschiedene Wassermenge im Wasserabscheider verfolgen und führt im allgemeinen zur Bildung der theoretischen Menge an Kondensatwasser.

An den Acetalisierungsschritt schließt sich eine Neutralisation des Katalysatoranteils des Reaktionsgemisches an, die die Hydrolyse des gebildeten Acetals verhindern soll. Zur Neutralisation sind Metallhydroxide und Metallalkoxide, insbesondere Natriumhydroxid, Kaliumhydroxid, Kalium-tert.-butylat, Natriummethylat oder methanolische Natriummethanolat-Lösung geeignet.

Die erfindungsgemäßen Acetale können ausgehend von den Hydroxyfettsäuren auf verschiedenen Wegen erzeugt werden.
Zum einen kann die Umsetzung der Hydroxyfettsäuren zu den Acetalen der Hydroxyfettsäureester oder Hydroxyfettsäureamide in zwei Schritten zunächst unter Veresterung oder Amidierung der Hydroxyfettsäure zum Ester oder Amid, Die angegebenen Werte für die Viskosität wurden bei 25°C und einer Scherrate von D = 10s⁻¹ mit einem Bohlin-Viskosimeter gemessen. Alle %-Angaben, soweit nicht anders angegeben, sind als Gew.-% zu verstehen.

### Beispiel 1:

### 12-Hydroxystearinsäure-2-ethylhexylesterformal (Eintopfverfahren) 13,15-Dioxa-12,16-di-n-hexyl-heptacosandisäure-di-ethylhexylester

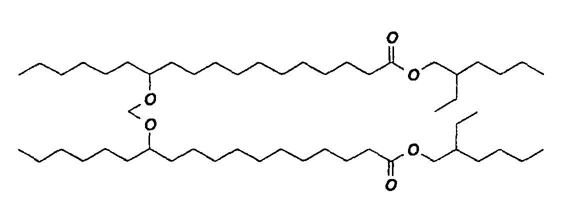

In einem 1 I-Vierhalskolben mit Wasserabscheider werden 156,2 g (0,50 mol) techn. 12-Hydroxystearinsäure, 65,1 g (0,50 mol) 2-Ethylhexanol und 0,86 g konz. Schwefelsäure (0,4 %) in 250 ml Cyclohexan vorgelegt und unter Stickstoffatmosphäre zum Rückfluß (80°C) erhitzt, bis die theoret. Menge Wasser abgeschieden ist (5 h).
Bei 80°C werden zu der rohen Esterlösung insgesamt 9,76 g (0,325 mol, 130 mol-%) Paraformaldehyd in 5 Portionen innerhalb 2 h eingetragen. Anschließend wird 2 h bei 80 °C nachkondensiert. Dabei wird die theoretische Menge Wasser (4,5 ml) im Wasserabscheider abgeschieden. Die Rohlösung wird abgekühlt, mit 4 g methanolischer Natriummethylat-Lösung auf pH 9 gestellt und unter Zusatz von Filtrierhilfsmittel filtriert. Das Lösungsmittel wird im Vakuum bei 110 °C/25 mm abdestilliert.
Ausbeute: 206,9 g (95,5 %) eines dünnflüssigen Öls.
Säurezahl 0,4 mg KOH/g, Viskosität 77 mPas.

### Beispiel 2:

### 12-Hydroxystearinsäure-methylesterformal 13,15-Dioxa-12,16-di-n-hexyl-heptacosandisäure-di-methylester dessen Zwischenisolierung und anschließender Acetalisierung erfolgen ( Beispiele 3, 4, 9, 10, 11). Im Falle der Acetalester kann die Durchführung dieser Schritte in einem Reaktionsgefäß in einer Eintopfreaktion erfolgen. Hierbei benutzt man das gleiche Reaktionsgefäß und den gleichen Katalysator für den Veresterungs- und den Acetalisierungsschritt (Beispiele 1, 5, 6, 8).

Alternativ dazu können Acetale von Hydroxyfettsäureestern und Hydroxyfettsäureamiden auch dadurch gewonnen werden, daß man zunächst aus der entsprechenden Hydroxyfettsäure, wie oben beschrieben, das Acetal gewinnt (Beispiel 12) und dieses im zweiten Schritt mit einem geeigneten Alkohol oder Amin zu dem entsprechenden Acetalester oder -amid umsetzt (Beispiel 13).

Eine weitere Möglichkeit bietet die Synthese eines Hydroxyfettsäureesters eines C₁-C₄-Alkohols wie Methanol, Ethanol, Propanol oder Butanol (z.B. 12-Hydroxystearinsäuremethylester), dessen anschließende Acetalisierung (Beispiel 2) zum entsprechenden Esteracetal und die darauffolgende Umesterung oder Umamidierung zum gewünschten Endprodukt. Diese Vorgehensweise ist insbesondere zur Synthese von Monoalkylamiden von Acetaldicarbonsäuren geeignet (Beispiel 14).

Die erfindungsgemäßen Acetale können als Basisöl für Metallbearbeitungsflüssigkeiten Verwendung finden. Ein besonderer Vorteil der Acetale ist, daß sie mit Mineralölfraktionen und Polyglykolethern teilweise mischbar sind, so daß die Acetale auch gemeinsam mit diesen Verbindungen eingesetzt werden können.

Die nachfolgenden Beispiele sollen die Erfindung näher erläutern, ohne sie zu beschränken:

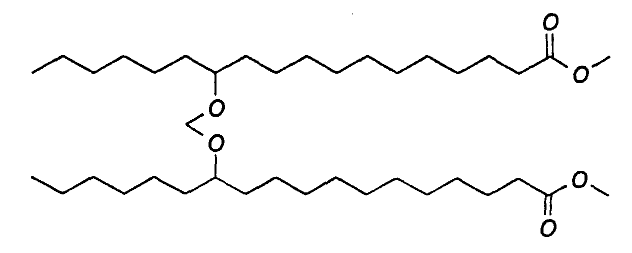

In einem 1 I-Vierhalskolben mit Wasserabscheider werden 178,2 g (0,50 mol) 12-Hydroxystearinsäuremethylester und 0,36 g
p-Toluolsulfonsäure-Monohydrat (0,2 %) in 250 ml Cyclohexan bei 80 °C gelöst. Die Lösung wird bei 87 °C Innentemperatur zum Rückfluß erhitzt, dann werden insgesamt 9,76 g (0,325 mol, 130 mol-%) Paraformaldehyd in 5 Portionen innerhalb 2 h eingetragen, wobei die theoretische Menge an Wasser abgeschieden wird. Anschließend wird 2 h bei 80 °C nachgerührt. Die Rohlösung wird mit 1,61 g 30 % methanolischer Natriummethylat-Lösung auf pH 9 gestellt und mit Filtrierhilfsmittel filtriert. Das Lösungsmittel wird im Vakuum bei 80 °C/30 mm abdestilliert.
Ausbeute: 179,0 g (98,8 %) eines trüben Öls, Säurezahl 2,0 mg KOH/g, Viskosität 113 mPas.

### Beispiel 3: (Vergleich)

### 12-Hydroxystearinsäure-oleylesterformal (zweistufiges Verfahren) 13,15-Dioxa-12,16-di-n-hexyl-heptacosandisäure-di-oleylester

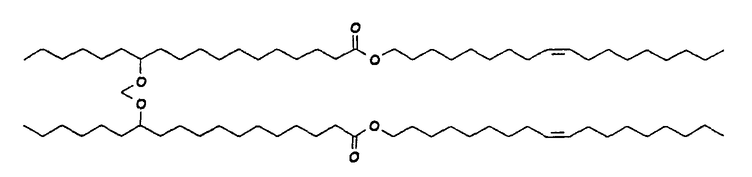

### a) 12-Hydroxystearinsäureoleylester

In einem 2 I-Vierhalskolben mit Wasserabscheider werden 500,0 g (1,610 mol) techn. 12-Hydroxystearinsäure, 436,8 g (1,64 mol, 102 %) techn. Oleylalkohol und 0,61 g konz. Schwefelsäure (0,02 %) in 200 ml Cyclohexan vorgelegt und unter Stickstoffatmosphäre zum Rückfluß (110 - 142 °C Innentemperatur) erhitzt, bis die theoret. Menge (28,8 ml) Wasser im Wasserabscheider abgeschieden ist (5 h). Das Lösungsmittel wurde bei 100 °C/20 mm abdestilliert. Ausbeute: 888,5 g (97,8 %) eines Feststoff, Säurezahl 1,7 mg KOH/g.

### b) 12-Hydroxystearinsäureoleylesterformal

In einem 2 I Vierhalskolben mit Wasserabscheider werden 868,0 g (1,48 mol) 12-Hydroxystearinsäureoleylester und 1,74 g p-Toluolsulfonsäure-Monohydrat (0,2 %) in 740 ml Cyclohexan bei 80 °C gelöst. Die Lösung wird bei 84 - 86 °C Innentemperatur zum Rückfluß erhitzt, dann werden insgesamt 28,9 g (0,962 mol, 130 mol-%) Paraformaldehyd in 5 Portionen innerhalb 2 h eingetragen, wobei die theoretische Menge an Wasser abgeschieden wird. Anschließend wird 2 h bei 80 °C nachgerührt. Die Rohlösung wird mit 4,53 g 30 %iger methanolischer Natriummethylat-Lösung auf pH 9 gestellt und mit Filtrierhilfsmittel filtriert. Das Lösungsmittel wird im Vakuum bei 110 °C/35 mm abdestilliert.
Ausbeute: 840,4 g (95,9 %) eines Öls, Säurezahl 0,9 mg KOH/g, Viskosität 519 mPas.

### Beispiel 4:

### 12-Hydroxystearinsäureisoamylesterformal 13,15-Dioxa-12,16-di-n-hexyl-heptacosandisäure-di-isoamylester

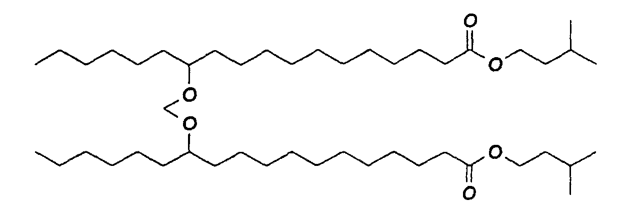

### a) 12-Hydroxystearinsäureisoamylester

In einem 2 I-Vierhalskolben mit Wasserabscheider werden 500,0 g (1,600 mol) techn. 12-Hydroxystearinsäure, 169,3 g (1,92 mol ) Isoamylalkohol und 1,25 g konz. Schwefelsäure (0,19 %) in 1200 ml Cyclohexan vorgelegt und unter Stickstoffatmosphäre zum Rückfluß (80 - 85 °C) erhitzt, bis die theoret. Menge (28,8 ml) Wasser im Wasserabscheider abgeschieden ist (10 h).
Das Lösungsmittel und der überschüssige Alkohol wird bei 100 °C/20 mm abdestilliert.
Ausbeute: 576,2 g (93,5 %) Feststoff, Schmp. 23 °C, Säurezahl 1,7 mg KOH/g.

### b) 12-Hydroxystearinsäureisoamylesterformal

In einem 1 I Vierhalskolben mit Wasserabscheider werden 186,3 g (0,50 mol) 12-Hydroxystearinsäureisoamylester und 0,37 g p-Toluolsulfonsäure-Monohydrat (0,2 %) in 250 ml Cyclohexan bei 80 °C gelöst. Die Lösung wird bei 90 - 92 °C zum Rückfluß erhitzt, dann werden insgesamt 9,76 g (0,325 mol, 130 mol-%) Paraformaldehyd in 5 Portionen innerhalb 2 h eingetragen, wobei 4,6 ml Wasser abgeschieden werden. Anschließend wird 2 h bei 80 °C nachgerührt. Die Rohlösung wird mit 1,88 g 30 %iger methanolischer Natriummethylat-Lösung auf pH 9 gestellt und mit Filtrierhilfsmittel filtriert. Das Lösungsmittel wird im Vakuum bei 110 °C/25 mm abdestilliert.
Ausbeute: 187,1 g (98,6 %) eines dünnflüssigen Öls, Säurezahl 0,4 mg KOH/g, Viskosität 71,3 mPas.

### Beispiel 5:

### 12-Hydroxystearinsäure-isotridecylesterformal (Eintopfverfahren) 13,15-Dioxa-12,16-di-n-hexyl-heptacosandisäure-di-isotridecylester

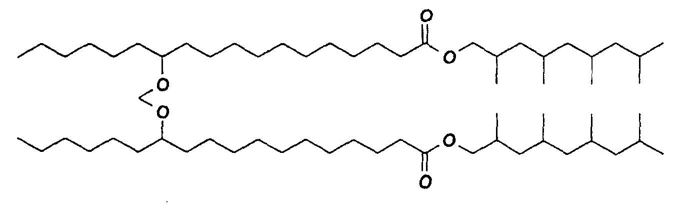

In einem 1 I-Vierhalskolben mit Wasserabscheider wurden 154,0 g (0,50. mol) techn. 12-Hydroxystearinsäure, 99,4 g (0,50 mol) Isotridecylalkohol, 250 ml Cyclohexan und 1,01 g (0,4 %) konz. Schwefelsäure vorgelegt und am Wasserabscheider zum Rückfluß erhitzt, dabei wurden innerhalb 9 h 9,0 ml Wasser abgeschieden. Zu dieser Lösung wurde bei 80 °C insgesamt 9,75 g (0,325 mol) Paraformaldehyd in fünf Portionen innerhalb 2 h eingetragen und 2 h bei dieser Temperatur nachgerührt. Das Reaktionsgemisch wurde auf Raumtemperatur abkühlen lassen, mit 4,0 g 30 %iger methanolischer Natriummethylatlösung auf pH 9 gestellt, unter Zusatz von Filtrierhilfsmittel filtriert und bei 100 °C/15 mb vom Lösungsmittel befreit.
Ausbeute: 246,0 g (96,5 %) eines orangen klaren Öls.
Viskosität (25 °C, Bohlin, 10s⁻¹) = 182 mPas.

### Beispiel 6:

### 12-Hydroxystearinsäure-methyltetraglykolesterformal (Eintopfverfahren) 13,15-Dioxa-12,16-di-n-hexyl-heptacosandisäure-di-methyltetraglykolester

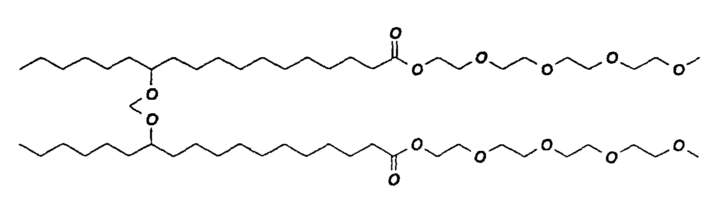

In einem 1 I Vierhalskolben mit Wasserabscheider werden 150,2 g (0,5 mol) techn. 12-Hydroxystearinsäure, 99,8 g (0,5 mol) Methyltetraglykol und 0,4 g konz. Schwefelsäure (0,16 %) in 250 ml Cyclohexan vorgelegt und unter Stickstoffatmosphäre zum Rückfluß (87 - 89 °C Innentemperatur) erhitzt, bis die theoret. Menge Wasser abgeschieden ist (16,5 h).
Bei 80 °C werden zu der rohen Esterlösung insgesamt 9,76 g (0,325 mol) Paraformaldehyd in 5 Portionen innerhalb 2 h eingetragen. Anschließend wird 2 h bei 80 °C nachgerührt. Dabei wird die theoretische Menge Wasser im Wasserabscheider abgeschieden. Die Rohlösung wird mit 5,80 g methanolischer Natriummethylat-Lösung auf pH 9,5 gestellt und unter Zusatz von Filtrierhilfsmittel filtriert. Das Lösungsmittel wird im Vakuum bei 110 °C/25 mm abdestilliert.
Ausbeute: 233,5 g (92,6 %) eines dünnflüssigen Öls.
Säurezahl 0,4 mg KOH/g, Viskosität 229.mPas.

### Beispiel 7:

### 12-Hydroxystearinsäure-tridecafluoroctylester-formal

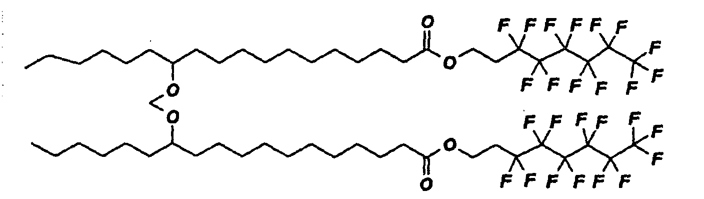

13,15-Dioxa-12,16-di-n-hexyl-heptacosandisäure-di-tridecafluoroctylester 158,3 g (0,50 mol) 12-Hydroxystearinsäure, 179,9 g (0,50 mol) 3,3,4,4,5,5,6,6,7,7,8,8,8-Tridecafluoroctanol und 1,35 g (0,4 %) Methansulfonsäure wurden in 250 ml Cyclohexan gelöst und bei 86 °C Innentemperatur am Wasserabscheider 9,5 ml Wasser abgetrennt. Innerhalb 2 h wurden in diese Lösung in 5 Portionen insgesamt 9,75 g (0,33 mol) Paraformaldehyd eingetragen und weitere 3 h gerührt, wobei insgesamt 4,7 ml Wasser abgeschieden wurden. Nach Abkühlen wurde mit 1,5 g Natriummethylat auf pH 9 gestellt und unter Zugabe von Filtrierhilfsmittel filtriert. Das Lösungsmittel wurde bei 100 °C/15 mb abdestilliert.
Ausbeute: 312,5 g eines trüben Öls, Verseifungszahl 95,4 mg KOH/g

### Beispiel 8: (Vergleich)

### 12-Hydroxystearinsäure-(Genapol O 050®)ester-formal

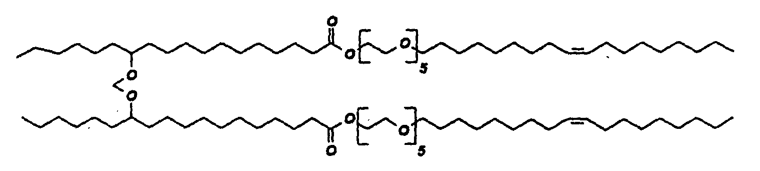

In einem 1 I Vierhalskolben mit Wasserabscheider wurden 158,3 g (0,50 mol) 12-Hydroxystearinsäure, 230,0 g (0,50 mol) Genapol O 050® (Oleylalkohol + 5 Ethylenoxid), und 1,55 g (0,4 %) Methansulfonsäure in 250 ml Cyclohexan gelöst und 16 h bei 92 °C Innentemperatur Wasser ausgekreist. Es ergab sich eine Restsäurezahl von 7,00 mg KOH/g. Innerhalb 2 h wurden dann bei 92 °C insgesamt 9,75 g (0,33 mol) Paraformaldehyd in 5 Portionen zudosiert und 2 h nachgerührt, dabei schied sich die theoretische Wassermenge ab. Nach Abkühlen wurde mit 2,0 g Natriummethylat auf pH 9 gestellt und in 500 ml Methanol gelöst, mit Filtrierhilfsmittel filtriert und bei 100 °C/15 mb vom Lösungsmittel befreit.
Ausbeute: 346,8 g rotbraunes, trübes Öl.
Verseifungszahl: 75,7 mg KOH/g.

### Beispiel 9:

### Ricinolsäure-methylesterformal (zweistufiges Verfahren) (Z,Z)-13,15-Dioxa-12,16-di-n-hexyl-heptacosa(9,18)diendisäure-di-methylester

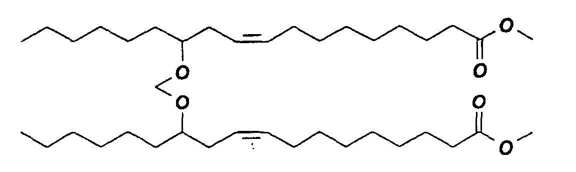

In einem 1 I Vierhalskolben mit Wasserabscheider werden 158,2 g (0,500 mol) Ricinolsäuremethylester und 0,32 g p-Toluolsulfonsäure-Monohydrat (0,2 %) in 250 ml Cyclohexan gelöst und zum Rückfluß (80 - 87 °C Innentemperatur) erhitzt. Anschließend werden 9,76 g (0,325 mol) Paraformaldehyd in 5 Portionen innerhalb 2 h eingetragen. Anschließend wird 2 h bei 80 °C nachgerührt. Dabei wird die theoretische Menge Wasser im Wasserabscheider abgeschieden. Die Rohlösung wird mit 2,4 g methanolischer
Natriummethylat-Lösung auf pH 9 gestellt und mit Filtrierhilfsmittel filtriert. Das Lösungsmittel wird im Vakuum bei 110 °C/25 mm abdestilliert.
Ausbeute: 155,5 g (96,5 %) eines farblosen Öls, Säurezahl 0,5 mg KOH/g, Viskosität 108 mPas.

### Beispiel 10:

### 12-Hydroxystearinsäure-morpholidformal 13,15-Dioxa-12,16-di-n-hexyl-heptacosandisäure-di-morpholid

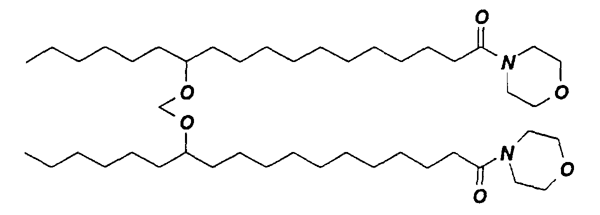

### a) 12-Hydroxystearinsäure-morpholid

314,3 g (1,00 mol) technische Hydroxystearinsäure und 87,1 g (1,00 mol) Morpholin werden vereinigt und auf 150 °C erhitzt, dabei destilliert ein Morpholin-Wasser Azeotrop ab. Es werden insgesamt weitere 104,5 g (1,20 mol) Morpholin innerhalb 20 h zugetropft und dabei jeweils das Reaktionswasser und das überschüssige Morpholin abdestilliert. Das überschüssige Morpholin wurde bei 170 °C/19 Torr abdestilliert; es verbleiben 377,0 g (97,5 %) eines bräunlichen, wachsartigen Feststoffes, Schmp. 36 - 38 °C, Säurezahl 3,3 mg KOH/g, Basenstickstoff 0,08 %.

### b) 12-Hydroxystearinsäure-morpholidformal

In einem 1 I Vierhalskolben mit Wasserabscheider werden 197,6 g (0,5 mol) des obigen 12-Hydroxystearinsäure-morpholids und 2,60 g p-Toluolsulfonsäure-Monohydrat in 250 ml Cyclohexan vorgelegt und unter Stickstoffatmosphäre zum Rückfluß (80 °C) erhitzt. Bei 80 °C werden zu der rohen Esterlösung insgesamt 9,76 g (0,325 mol, 130 %) Paraformaldehyd in 5 Portionen innerhalb 2 h eingetragen. Anschließend wird 2 h bei 80 °C nachgerührt. Dabei wird die theoretische Menge Wasser im Wasserabscheider abgeschieden. Die Rohlösung wird mit 4 g 30 %iger methanolischer Natriummethylat-Lösung auf pH 9 gestellt und mit Filtrierhilfsmittel filtriert. Das Lösungsmittel wird im Vakuum bei 110 °C/25 mm abdestilliert.
Ausbeute: 172,1 g (98,0 %) eines trüben Öls.
Säurezahl 1,7 mg KOH/g, Viskosität 798 mPas.

### Beispiel 11:

### 12-Hydroxystearinsäure-methoxypropylamid-formal 13,15-Dioxa-12,16-di-n-hexyl-heptacosandisäure-di-3-methoxypropylamid

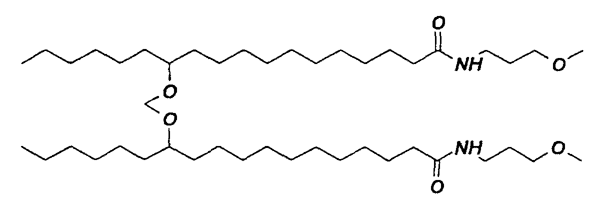

### a) 12-Hydroxystearinsäure-methoxypropylamid

500,0 g (1,60 mol) 12-Hydroxystearinsäure und 157,0 g (1,76 mol, 110 %) 3-Methoxypropylamin werden vereinigt und auf 150 °C erhitzt, dabei destilliert innerhalb 6,5 h ein Amin-Wasser-Gemisch über. Es werden weiter 15,7 g (0,17 mol, 10 %) 3-Methoxypropylamin zudosiert und bei 190 °C weitere 6 h abdestilliert. Das überschüssige Amin wird bei 180 °C/20 mm abdestilliert. Es verbleiben 579,0 g (94,3 %) eines Feststoffes, Schmp. 94 - 96 °C, Säurezahl 1,31 mg KOH/g, Basenstickstoff < 0,1 %.

### b) 12-Hydroxystearinsäure-methoxypropylamid-formal

In einem 1 I Vierhalskolben mit Wasserabscheider werden 207,3 g (0,50 mol) des obigen 12-Hydroxystearinsäure-methoxypropylamids und 0,41 g p-Toluolsulfonsäure-Monohydrat (0,2 %) in 250 ml Cyclohexan vorgelegt und unter Stickstoffatmosphäre zum Rückfluß (80 °C) erhitzt. Bei 84 °C Innentemperatur werden zu der rohen Esterlösung insgesamt 9,76 g (0,325 mol) Paraformaldehyd in 5 Portionen innerhalb 2 h eingetragen. Anschließend wird 2 h bei 80 °C nachgerührt. Dabei wird die theoretische Menge Wasser im Wasserabscheider abgeschieden. Die Rohlösung wird mit 2,35 g methanolischer Natriummethylat-Lösung auf pH 9 gestellt und mit Filtrierhilfsmittel filtriert. Das Lösungsmittel wird im Vakuum bei 110 °C/30 mm abdestilliert.
Ausbeute: 193,8 g (92,1 %) wachsartiger Feststoff.
Säurezahl O mg KOH/g.

### Beispiel 12:

### 12-Hydroxystearinsäureformal 13,15-Dioxa-12,1 6-di-n-hexyl-heptacosandisäure

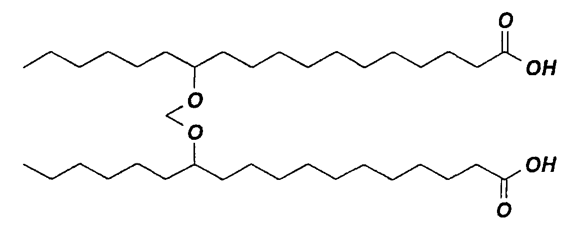

150,2 g (0,50 mol) 12-Hydroxystearinsäure und 0,64 g konz. Schwefelsäure werden in einem 1 I Vierhalskolben mit Wasserabscheider in 250 ml n-Hexan vorgelegt und auf Rückflußtemperatur erhitzt. Zu der entstandenen Lösung werden insgesamt 9,76 g (0,325 mol) Paraformaldehyd in 5 Portionen innerhalb 2 h eingetragen. Anschließend wird 2 h bei 68 °C nachgerührt. Dabei wird die theoretische Menge Wasser am Wasserabscheider abgeschieden. Nach Abkühlen auf 30 °C wird die Rohlösung mit 2,60 g 30 %iger methanolischer Natrium-methylat-Lösung versetzt und mit Filtrierhilfsmittel filtriert. Das Lösungsmittel wird im Vakuum bei 60 °C/15 mbar abdestilliert.
Ausbeute: 155,4 g ( wachsartiger Feststoff)
Säurezahl: 141 mg KOH/g
Verseifungszahl: 183,9 mg KOH/g

### Beispiel 13:

### 12-Hydroxystearinsäure-3-methoxypropylamidformal 13,15-Dioxa-12,16-di-n-hexyl-heptacosandisäure-di-3-methoxypropylamid

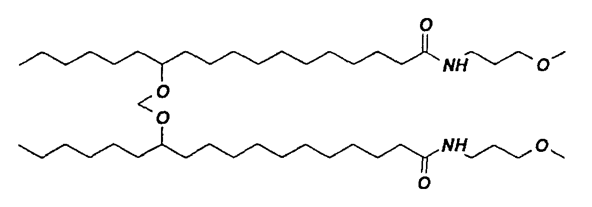

Das nach Beispiel 12 hergestellte Hydroxystearinsäureformal wird mit 44,6 g (0,50 mol) 3-Methoxypropylamin versetzt und bei 133 °C Innentemperatur in Cyclohexan unter azeotroper Wasserabscheidung 17 h erhitzt. Nach Abdestillieren des Lösungsmittels wird in 500 ml Methanol gelöst und und bei 80 °C filtriert. Das Methanol wird bei bei 100 °C/15 mbar abdestilliert.
Ausbeute: 161,6 g hellbraunes Wachs; Amidstickstoff: 2,69 %
Schmelzpunkt: 82 - 87 °C

### Beispiel 14:

### 12-Hydroxystearinsäure-3-methoxypropylamidformal 13,15-Dioxa-12,16-di-n-hexyl-heptacosandisäure-di-3-methoxypropylamid durch Umamidierung des Methylesters

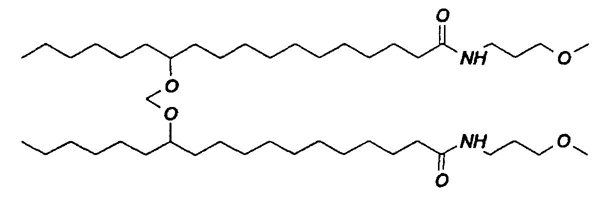

In einem 500 ml Vierhalskolben mit kurzer Vigreux-Kolonne und aufgesetztem Kolonnenkopf wurden 159,2 g (0,50 mol) 12-Hydroxystearinsäuremethylesterformal (vgl. Beispiel 2) und 49,0 g 3-Methoxypropylamin vereinigt und die Reaktionsmischung 10 h zum Rückfluß erhitzt. In 3,5 h wurden 16 ml Destillat abgenommen. Es wurden nochmals 22,9 g (0,25 mol) 3-Methoxypropylamin zugegeben und innerhalb 7,5 h weitere 3,5 ml Destillat abgenommen. Das Reaktionsprodukt wurde anschließend bei 140 °C/15 mb von überschüssigem 3-Methoxypropylamin befreit.
Ausbeute: 181,7 g beigefarbenes Wachs, Schmp. 58-71 °C, Amidstickstoff 3,44 %.

### Beispiel 15:

### 12-Hydroxystearinsäure-methyltetraglykol/2-ethylhexanolmischesterformal

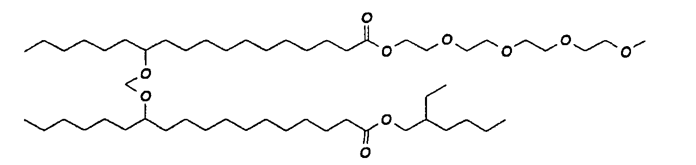

In einem 1 I Vierhalskolben mit Wasserabscheider werden 156,2 g (0,50 mol) techn. 12-Hydroxystearinsäure, 32,6 g (0,25 mol) 2-Ethylhexanol, 49,9 g (0,25 mol) Methyltetraglykol und 0,96 g konz. Schwefelsäure (0,4 %) in 250 ml Cyclohexan vorgelegt und unter Stickstoffatmosphäre zum Rückfluß (80 °C) erhitzt, bis die theoretische Menge Wasser abgeschieden ist (7,5 h). Bei 80 °C werden zu der rohen Esterlösung insgesamt 9,76 g (0,325 mol, 130 mol-%) Paraformaldehyd in 5 Portionen innerhalb 2 h eingetragen. Anschließend wird 2 h bei 80 °C nachkondensiert. Dabei wird die theoretische Menge Wasser (4,5 ml) im Wasserabscheider abgeschieden. Die Rohlösung wird abgekühlt, mit 3,5 g methanolischer Natriummethylat-Lösung auf pH 9 gestellt und unter Zusatz von Filtrierhilfsmittel filtriert. Das Lösungsmittel wird im Vakuum bei 110 °C/25 mm abdestilliert.
Ausbeute: 217,7 g (93,6 %) eines Öls.
Säurezahl 1,4 mg KOH/g, Viskosität 756 mPas.

### Beispiel 16:

### Milchsäure-2-ethylhexylester-formal

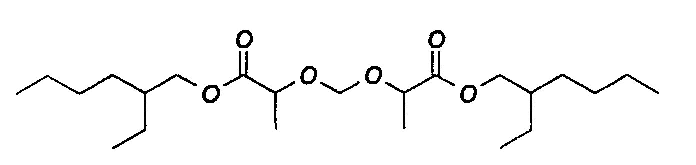

In einem 500 ml Vierhalskolben mit Wasserabscheider wurden 182,0 g (0,90 mol) Milchsäure-2-ethylhexylester und 0,38 g (0,2 %) konz. H₂SO₄ in 190 g Cyclohexan vorgelegt, zum Rückfluß erhitzt (90 °C) und in 5 Portionen innerhalb 4 h insgesamt 17,60 g (0,586 mol) Paraformaldehyd zugegeben, anschließend wurde 2 h bei 90 °C nachgerührt. Insgesamt wurden 9,2 ml Wasser abgeschieden. Nach dem Abkühlen wurde mit 1,88 g Kalium-tert.-butylat auf pH 8 gestellt und das Lösungsmittel bei 70 °C/25 mm abdestilliert.
Ausbeute: 186,6 g gelbes Öl, Säurezahl 0,23 mg KOH/g, Verseifungszahl 248,8 mg KOH/g.

### Beispiel 17:

### 6-Hydroxyhexansäure-2-ethylhexylester-formal

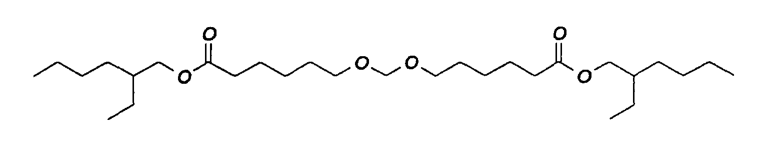

57,1 g (0,50 mol) Caprolacton, 65,1 g (0,50 mol) 2-Ethylhexanol und 0,25 g konz. Schwefelsäure wurden vereinigt und 5 h auf 100 °C erhitzt. Die Reaktionsmischung wurde in 250 ml Cyclohexan gelöst und am Wasserabscheider zum Rückfluß erhitzt. In Abständen von jeweils 30 min wurde in 5 Portionen insgesamt 9,75 g (0,325 mol) Paraformaldehyd eingetragen, wobei 4,5 ml Wasser abgeschieden wurden. Anschließend wurde 2 h bei 82 °C nachgerührt. Nach dem Abkühlen wurde mit 1,55 g 30 %iger Natriummethylatlösung auf pH 9 gestellt, mit Celite filtriert und bei 80 °C/15 mb zur Gewichtskonstanz eingeengt. Ausbeute: 121,2 g hellgelbes, klares Öl, Säurezahl <0,1 mg KOH/g, Verseifungszahl 230 mg KOH/g.

### Beispiel 18:

### 12-Hydroxystearinsäure-methylester-p-nitrobenzaldehydacetal

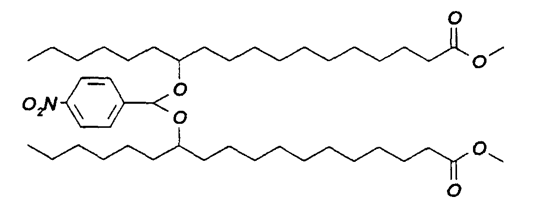

157,3 g (0,50 mol) 12-Hydroxystearinsäuremethylester, 39,7 g (0,263 mol) 4-Nitrobenzaldehyd und 0,40 g (0,2 %) konz. H₂SO₄ wurden in 350 ml Cyclohexan vereinigt und 20 h auf 83 °C erhitzt, wobei 3,0 ml Wasser abgeschieden wurden. Die Reaktionsmischung wurde mit festem Natriummethylat auf pH 9 gestellt und unter Zusatz von Filtrierhilfsmittel filtriert. Das Lösungsmittel wurde bei 100 °C/15 mb abgezogen.
Ausbeute: 193,3 g eines orangebraunen Öls.

### Beispiel 19:

### 12-Hydroxystearinsäure-(polyglykol M 750)-esterformal (Eintopfverfahren)

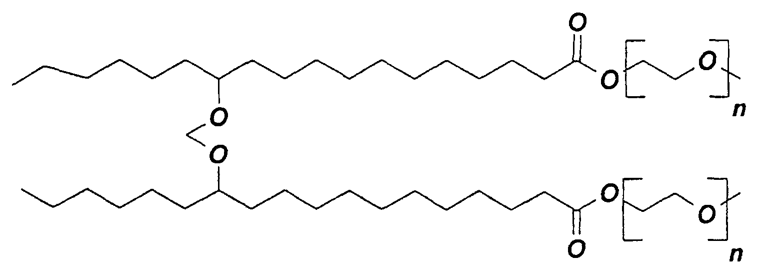

123 g (0,40 mol) 12-Hydroxystearinsäure, 307 g (0,40 mol) Polyglykol M 750 (Methylpolyglykol) und 1,72 g (0,4 %) Methansulfonsäure wurden in 156 g Cyclohexan vereinigt und 37 h auf 84-98 °C am Wasserabscheider erhitzt, wobei die theoretische Wassermenge ausgekreist wurde. Anschließend wurden 7,81 g (0,26 mol) Paraformaldehyd in 5 Portionen im Abstand von 30 min zugegeben und anschließend noch weitere 2 h bei Rückflußtemperatur erhitzt. Es wurde mit 3,50 g 30 % methanolischer Natriummethylatlösung auf pH 9 gestellt, mit Filtrierhilfsmittel filtriert und das Filtrat bei max. 100 °C/25 mbar vom Lösungsmittel befreit.
Ausbeute: 405 g eines gelbbraunen Wachses, Säurezahl 0,5 mg KOH/g, Viskosität (Bohlin, 40 °C, 10 s⁻¹) = 125 mPas.

## Patentansprüche

1. Acetale der allgemeinen Formel R³CO-X-O-C(R¹R²)-O-Y-COR⁴ worin
R¹, R² unabhängig voneinander Wasserstoff, einen substituierten oder unsubstituierten C₁-C₁₂-Alkyl-, C₆-C₁₂-Aryl- oder C₇-C₁₂-Aralkylrest,
X, Y unabhängig voneinander einen verzweigten oder unverzweigten, substituierten oder unsubstituierten C₁₅-C₃₀-Alkylenrest oder einen verzweigten oder unverzweigten, substituierten oder unsubstituierten C₁₅-C₃₀-Alkenylenrest,
R³, R⁴ unabhängig voneinander -OH, -OM, -OR⁵, -NH₂, -NHR⁶ oder -N R⁷ R⁸ bedeuten, wobei
-M ein Alkali-, Erdalkali- oder ein Ammoniumion der Formel HN⁺R⁹R¹⁰R¹¹ oder Mischungen davon bedeutet, wobei
R⁹ bis R¹¹ unabhängig voneinander Wasserstoff, C₁-C₁₀-Alkyl oder C₁-C₁₀-Hydroxyalkyl bedeuten und wobei
R⁵ bis R⁸ unabhängig voneinander einen C₁-C₂₀₀-Alkyl-, einen C₆-C₅₀-Aryl- oder einen C₇-C₅₀-Aralkylrest bedeuten, und wobei
R⁵ bis R⁸ mit Heteroatomen substituiert oder durch Heteroatome unterbrochen sein können und wobei
R⁷ und R⁸ gemeinsam einen Ring von 4 bis 10 Atomen bilden können.

2. Acetale gemäß Anspruch 1, worin R¹ und R² unabhängig voneinander Wasserstoff oder einen C₁-C₆-Alkylrest, insbesondere Wasserstoff, bedeuten.

3. Acetale gemäß Anspruch 1 oder 2, worin R³ und R⁴ unabhängig voneinander -OR⁵ oder -NHR⁶ oder -NR⁷R⁸ bedeuten.

4. Acetale gemäß einem der Ansprüche 1 bis 3, worin R⁵ bis R⁸ unabhängig voneinander einen C₁-C₁₈-, insbesondere einen C₅- C₁₃-Alkylrest bedeuten.

5. Acetale gemäß einem der Ansprüche 1 bis 4, worin R⁷ und R⁸ gemeinsam einen Ring von 6 Atomen im Ringgerüst bilden.

6. Verfahren zur Herstellung von Acetalen gemäß Anspruch 1, **dadurch gekennzeichnet, daß** eine Hydroxycarbonsäure oder deren Derivate oder Mischungen davon mit einem Aldehyd unter Säurekatalyse zur Reaktion gebracht werden.

7. Verfahren zur Herstellung von Acetalen gemäß Anspruch 1, **dadurch gekennzeichnet, daß** eine Hydroxycarbonsäure mit einem Aldehyd unter Säurekatalyse zur Reaktion gebracht und das entstandene Acetal derivatisiert wird.

8. Verfahren zur Herstellung von Acetalen gemäß Anspruch 1, **dadurch gekennzeichnet, daß** man den Ester aus Hydroxyfettsäure und C₁-C₄-Alkohol acetalisiert und dann umestert oder umamidiert.

9. Verfahren gemäß Anspruch 6 oder 7, **dadurch gekennzeichnet, daß** die Umsetzung bei 40-140°C mit 0,01-2 Gew.-% konzentrierter Schwefelsäure oder Methansulfonsäure bezüglich des eingesetzten Hydroxyfettsäurederivats durchgeführt wird, gefolgt von einem Neutralisationsschritt.

10. Verfahren gemäß einem der Ansprüche 6 bis 9, **dadurch gekennzeichnet, daß** 12-Hydroxystearinsäure oder Ricinolsäure und Formaldehyd oder Paraformaldehyd eingesetzt werden.

11. Verwendung von Acetalen gemäß den Ansprüchen 1 bis 5 als Basisöl für Metallbearbeitungsflüssigkeiten.

## Claims

1. An acetal of the formula R³CO-X-O-C(R¹R²)-O-Y-COR⁴, in which
R¹, R² independently of one another are hydrogen, a substituted or unsubstituted C₁-C₁₂-alkyl radical, a substituted or unsubstituted C₆-C₁₂-aryl radical or a substituted or unsubstituted C₇-C₁₂-aralkyl radical,
X, Y independently of one another are a branched or unbranched, substituted or unsubstituted C₁₅-C₃₀-alkylene radical or a branched or unbranched, substituted or unsubstituted C₁₅-C₃₀-alkenylene radical,
R³, R⁴ independently of one another are -OH, -OM, -OR⁵, -NH₂, -NHR⁶ or NR⁷R⁸, where
-M is an alkali metal ion, an alkaline earth metal ion or an ammonium ion of the formula HN⁺R⁹R¹⁰R¹¹ or mixtures thereof, where
R⁹ to R¹¹ ¹ independently of one another are hydrogen, C₁-C₁₀-alkyl or C₁-C₁₀-hydroxyalkyl and where
R⁵ to R⁸ independently of one another are a C₁-C₂₀₀-alkyl radical, a C₆-C₅₀-aryl radical or a C₇-C₅₀-aralkyl radical, and where
R⁵ to R⁸ can be substituted by heteroatoms or interrupted by heteroatoms and where
R⁷ and R⁸ together can form a ring of 4 to 10 atoms.

2. An acetal as claimed in claim 1, in which
R¹ and R² independently of one another are hydrogen or a C₁-C₆-alkyl radical, in particular hydrogen.

3. An acetal as claimed in claim 1 or 2, in which
R³ and R⁴ independently of one another are -OR⁵ or -NHR⁶ or -NR⁷R⁸.

4. An acetal as claimed in one of claims 1 to 3, in which
R⁵ to R⁸ independently of one another are a C₁-C₁₈-, in particular a C₅-C₁₃-, alkyl radical.

5. An acetal as claimed in one of claims 1 to 4, in which
R⁷ and R⁸ together form a ring of 6 atoms in the ring structure.

6. A process for preparing acetals as claimed in claim 1, which comprises reacting a hydroxycarboxylic acid or its derivatives or mixtures thereof with an aldehyde under acid catalysis.

7. The process for preparing acetals as claimed in claim 1, wherein
a hydroxycarboxylic acid is reacted with an aldehyde under acid catalysis and the resulting acetal is derivatized.

8. The process for preparing acetals as claimed in claim 1, wherein
the ester of a hydroxy fatty acid and C₁-C₄-alcohol is acetalized and is then transesterified or transamidated.

9. The process as claimed in claim 6 or 7, wherein the reaction is carried out at 40-140°C with 0.01-2% by weight of concentrated sulfuric acid or methanesulfonic acid with respect to the hydroxy fatty acid derivative used, followed by a neutralization step.

10. The process as claimed in one of claims 6 to 9,
wherein 12-hydroxystearic acid or ricinoleic acid and formaldehyde or paraformaldehyde are used.

11. The use of acetals as claimed in any of claims 1 to 5 as basis oil for metal machining fluids.

## Revendications

1. Acétals de formule générale R³CO-X-O-C(R¹R²)-O-Y-COR⁴ , dans laquelle
R₁, R₂ indépendamment l'un de l'autre, représentent l'hydrogène, un reste alkyle en C₁-C₁₂, aryle en C₆-C₁₂ ou aralkyle en C₇-C₁₂, substitué ou non substitué,
X, Y indépendamment l'un de l'autre, représentent un reste alkylène en C₁₅-C₃₀, substitué ou non substitué, ramifié ou non ramifié ou un reste alcénylène en C₁₅-C₃₀, substitué ou non substitué, ramifié ou non ramifié,
R₃, R₄ indépendamment l'un de l'autre, représentent -OH, -OM, -OR⁵, -NH₂, -NHR₆ ou -NR⁷R⁸, où
-M représente un ion alcalin, alcalino-terreux ou ammonium de formule HN⁺R⁹R¹⁰R¹¹ ou des mélanges de ceux-ci, où
R⁹ à R¹¹ indépendamment l'un de l'autre, représentent l'hydrogène, alkyle en C₁-C₁₀ ou hydroxyalkyle en C₁-C₁₀ et où
R⁵ à R⁸ indépendamment l'un de l'autre, représentent un reste alkyle en C₁-C₂₀₀, un reste aryle en C₆-C₅₀ ou un reste aralkyle en C₇-C₅₀, et où
R⁵ à R⁸ peuvent être substitués par des hétéroatomes ou interrompus par des hétéroatomes et où
R⁷ et R⁸ peuvent former ensemble un cycle à 4 à 10 atomes.

2. Acétals selon la revendication 1, dans lesquels R¹ et R², indépendamment l'un de l'autre, représentent l'hydrogène ou un reste alkyle en C₁-C₆, en particulier l'hydrogène.

3. Acétals selon la revendication 1 ou 2, dans lesquels R³ et R⁴, indépendamment l'un de l'autre, représentent -OR⁵ ou -NHR⁶ ou -NR⁷R⁸.

4. Acétals selon l'une des revendications 1 à 3, dans lesquels R⁵ à R⁸, indépendamment l'un de l'autre, représentent un reste alkyle en C₁-C₁₈, en particulier en C₅-C₁₃.

5. Acétals selon l'une des revendications 1 à 4, dans lesquels R⁷ et R⁸ forment ensemble un cycle à six atomes dans la structure cyclique.

6. Procédé pour la préparation d'acétals selon la revendication 1, **caractérisé en ce qu'**un acide hydroxycarboxylique ou ses dérivés ou mélanges de ceux-ci sont mis à réagir avec un aldéhyde sous catalyse acide.

7. Procédé pour la préparation d'acétals selon la revendication 1, **caractérisé en ce qu'**un acide hydroxycarboxylique est mis à réagir avec un aldéhyde sous catalyse acide et **en ce que** l'acétal résultant est transformé en dérivé.

8. Procédé pour la préparation d'acétals selon la revendication 1, **caractérisé en ce que** l'on acétalise l'ester d'acide gras hydroxylé et d'alcool en C₁-C₄ puis l'on transestérifie ou transamidifie.

9. Procédé selon la revendication 6 ou 7, **caractérisé en ce que** la réaction est effectuée à 40-140°C avec 0,01 à 2 % en poids d'acide sulfurique concentré ou d'acide méthanesulfonique, par rapport au dérivé d'acide gras hydroxylé utilisé, en faisant suivre d'une étape de neutralisation.

10. Procédé selon l'une des revendications 6 à 9, **caractérisé en ce que** l'acide 12-hydroxystéarique ou l'acide ricinoléique et le formaldéhyde ou le paraformaldéhyde sont utilisés.

11. Utilisation des acétals selon les revendications 1 à 5 comme huile de base pour des liquides de travail des métaux.
